# EUROPÄISCHE PATENTANMELDUNG

(11) **EP 1 645 269 A1**
(43) Veröffentlichungstag der Anmeldung: **12.04.2006**
(21) Anmeldenummer: 05019276.4
(22) Anmeldetag: 06.09.2005
(51) Int. Cl.: A61K 9/51, A61K 9/16

(54) **Verfahren und Anlage zur Herstellung von Mikropartikeln oder Nanopartikeln, Verwendung der Nanopartikel und damit hergestellte Suspension oder Emulsion**

(30) Priorität: 15.09.2004 DE 102004045056; 13.10.2004 DE 102004049850
(71) Anmelder: Cognis IP Management GmbH, 40589 Düsseldorf (DE)
(72) Erfinder: Schaber, Karlheinz, Prof. Dr., 76316 Malsch (DE); Sinanis, Sokratis, 76131 Karlsruhe (DE); Mall-Geissle, Susanne, 76768 Berg/Pfalz (DE)

(57) **Zusammenfassung**

Zur Herstellung von Mikropartikeln oder Nanopartikeln aus einer oder mehreren Substanzen, insbesondere aus Lipiden, überführt man die Substanz in die Gasphase, die man dann komprimiert und gleichzeitig abkühlt. Die Mikro- bzw. Nanopartikel scheidet man aus dem erzeugten Aerosol ab. Aus schwerflüchtigen Substanzen sind Nanopartikel mit einer hohen Partikelanzahlkonzentration herstellbar, so dass ein nachfolgendes Aufkonzentrieren nicht notwendig oder auf einfache Weise durchzuführen ist. Die Herstellung erfolgt unter milden Temperatur- und Druckbedingungen und mit geringem technischem Aufwand hinsichtlich der mechanischen Anforderungen der Anlage, der Apparatekosten und deren Wartung.

Es wird vorgeschlagen, die Zusammenfassung ohne Zeichnung zu veröffentlichen.

## Beschreibung

Die Erfindung betrifft ein Verfahren zur Herstellung von Mikropartikeln oder Nanopartikeln aus einer oder mehreren Substanzen, insbesondere aus Lipiden, wobei die Herstellung von Nanopartikeln bevorzugt ist.

Unter Nanopärtikeln sind hier insbesondere Stoffe mit einem mittleren Teilchendurchmesser im Bereich unterhalb 1 µm, vorzugsweise von 5 bis 700 nm, ganz besonders bevorzugt im Bereich von 10 bis 500 nm zu verstehen. Die Größenangaben sind zu verstehen als Durchmesser in Richtung der größten Längenausdehnung der Teilchen. Bei der Herstellung der Nanopartikel erhält man stets Teilchen mit einer Größe, die einer Verteilungskurve folgt. Zur experimentellen Bestimmung der Teilchengröße kann beispielsweise die dem Fachmann bekannte Methode der dynamischen Lichtstreuung angewandt werden.

Die Mikropartikel haben vorzugsweise einen mittleren Teilchendurchmesser von 1 bis 20 µm, insbesondere von etwa 1 bis 5 µm.

Unter dem Begriff "Substanzen" sind hier insbesondere feste Stoffe zu verstehen, welche einen Schmelzpunkt oder Schmelzbereich zwischen 25 °C und 300 °C haben. Es kann sich bei den genannten Substanzen aber auch um bei Raumtemperatur flüssige Stoffe handeln. Es kommen als Substanzen sowohl chemisch einheitliche Stoffe als auch Gemische in Betracht.

Die erfindungsgemäß eingesetzten Substanzen sind beispielsweise solche, welche einen industriellen Einsatz finden in der Kosmetik, der Pharmazie, in Nahrungsmitteln, in Wasch- und Reinigungsmitteln, in Klebstoffen, in der Oberflächenbehandlung, in Hygieneprodukten sowie in der Landwirtschaft. Insbesondere seien die im Bereich der Kosmetik eingesetzten Substanzen wie beispielweise UV-Lichtschutzfilter, Farbstoffe, Riechstoffe, Emulgatoren, Wachse, Rückfetter, Antioxidantien, Deodorantien und Antitranspirantien genannt. Weiterhin seien als bevorzugte Substanzen pharmakologisch aktive Substanzen zu nennen, die in der Kosmetik, Dermatologie und Pharmazie als Wirkstoff eingesetzt werden. Eine weitere Anwendung der Teilchen liegt im Bereich der Lebensmitteltechnologie. Dies gilt sowohl für Komponenten von Nahrungsmitteln als auch für Nahrungsergänzungsmittel. Besonders bevorzugt werden als Substanzen im Sinne des erfindungsgemäßen Herstellungsverfahrens lipophile Substanzen, im weiteren Lipide genannt, verwendet. Zu dieser Stoffklasse gehören unter anderem alle Fettsäure- und Fettalkohol-Derivate mit fettähnlichen Eigenschaften und/oder mit oberflächenaktiven Eigenschaften.

Erfindungsgemäß werden vorzugsweise Nanopartikel aus schwerflüchtigen Substanzen, vorwiegend Feststoffen, hergestellt.

Im Folgenden wird ein Überblick über die Herstellung von Nanopartikeln nach dem Stand der Technik gegeben:
Ein Verfahren zur Herstellung von Nanoteilchen durch rasche Entspannung von überkritischen Lösungen (Rapid Expansion of Supercritical Solutions RESS) ist beispielsweise aus dem Aufsatz von S. Chihlar, M. Türk und K. Schaber in Proceedings World Cöngress on Particle Technology 3, Brighton, 1998 bekannt. In einer bevorzugten Ausführungsform der Erfindung erhält man Nanopartikel, indem man
a) die Ausgangsstoffe unter überkritischen oder nahekritischen Bedingungen in einem geeigneten Lösungsmittel löst,
b) die fluide Mischung über eine Düse in ein Vakuum, ein Gas oder eine Flüssigkeit entspannt, und
c) das Lösemittel dabei gleichzeitig verdampft.
Um zu verhindern, dass die Nanopartikel wieder zusammenbacken, empfiehlt es sich, die Ausgangsstoffe in Gegenwart geeigneter Schutzkolloide oder Emulgatoren zu lösen, wobei Emulgatoren ebenfalls Schutzkolloide sind, und/oder die kritischen Lösungen in wässrige und/oder alkoholische Lösungen der Schutzkolloide bzw. Emulgatoren oder aber in kosmetische Öle zu entspannen, welche ihrerseits wieder gelöste Emulgatoren und/oder Schutzkolloide enthalten können. Geeignete Schutzkolloide sind dabei z. B. Gelatine, Casein, Gummi arabicum, Lysalbinsäure, Stärke sowie Polymere, wie etwa Polyvinylalkohole, Polyvinylpyrrolidone Polyalkylenglycole und Polyacrylate. Die bevorzugten Nanopartikel sind also die, die von einem Schutzkolloid und/oder einem Emulgator ummantelt vorliegen. Üblicherweise werden die Schutzkolloide oder Emulgatoren in Mengen von 0,1 bis 20, vorzugsweise 5 bis 15 Gew.-% - bezogen auf die feinteiligen Substanzen - eingesetzt.

Ein weiteres bekanntes Verfahren zur Herstellung der nanoskaligen Teilchen bietet die Evaporationstechnik. Hierbei werden die Ausgangsstoffe zunächst in einem geeigneten organischen Lösungsmittel (z. B. Alkane, pflanzliche Öle, Ether, Ester, Ketone, Acetale und dergleichen) gelöst. Anschliessend werden die Lösungen derart in Wasser oder einem anderen Nicht-Lösungsmittel, gegebenenfalls in Gegenwart einer darin gelösten oberflächenaktiven Verbindung gegeben, dass es durch die Homogenisierung der beiden nicht miteinander mischbaren Lösungsmittel zu einer Ausfällung der Nanoteilchen kommt, wobei das organische Lösungsmittel vorzugsweise verdampft. Anstelle einer wässrigen Lösung können auch O/W-Emuisionen bzw. O/W-Mikroemulsionen eingesetzt werden. Als oberflächenaktive Verbindungen können die genannten Emulgatoren und Schutzkolloide verwendet werden.

Eine weitere Möglichkeit zur Herstellung von Nanoteilchen besteht in dem sogenannten GAS-Verfahren (Gas Anti Solvent Recrystallization). Das Verfahren nutzt ein hochkomprimiertes Gas oder überkritisches Fluid (z. B. Kohlendioxid) als Nicht-Lösungsmittel zur Kristallisation von gelösten Stoffen. Die verdichtete Gasphase wird in die Primärlösung der Ausgangsstoffe eingeleitet und dort absorbiert, wodurch sich das Flüssigkeitsvolumen vergrössert, die Löslichkeit abnimmt und feinteilige Partikel ausgeschieden werden.

Bekannt ist auch das PCA-Verfahren (Precipitation with a Compressed Fluid Anti-Solvent). Hier wird die Primärlösung der Ausgangsstoffe in ein überkritisches Fluid eingeleitet, wobei sich feinstverteilte Tröpfchen bilden, in denen Diffusionsvorgänge ablaufen, so dass eine Ausfällung feinster Partikel erfolgt.

Beim PGSS-Verfahren (Particles from Gas Saturated Solutions) werden die Ausgangsstoffe durch Aufpressen von Gas (z. B. Kohlendioxid oder Propan) aufgeschmolzen. Druck und Temperatur erreichen nahe- oder überkritische Bedingungen. Die Gasphase löst sich im Feststoff und bewirkt eine Absenkung der Schmelztemperatur, der Viskosität und der Oberflächenspannung. Bei der Expansion durch eine Düse kommt es durch Abkühlungseffekte zur Bildung feinster Teilchen.

Im Stand der Technik werden Nano- und Mikropartikel außerdem erzeugt durch
- lnertgas-Kondensation/-Desublimation, nämlich durch Verdunstung/Sublimation in einen Trägergasstrom bei Normaldruck und anschließende Übersättigung des Trägergas-Dampf-Gemisches durch starke Kühlung bzw. Vermischen mit kaltem Gas. Dabei erfolgt die Partikelbildung durch Kondensation/Desublihlation (S. Mall-Gleissle, K.-H. Schaber: Chem-Ing.-Techn. (75) 2003,S. 1621-1624).
- Chemische Reaktion
- Mechanisches Zerstäuben von Flüssigkeiten
- Erzeugung einer Emulsion/Dispersion durch mechanische Zerkleinerung (z. B. Hochdruckhomogehisieren, Mahlen).

Zur Stabilisierung der mit Hilfe einer überkritischen Lösung hergestellten organischen Nanopartikel ist es bekannt, die überkritische Lösung direkt in eine wässrige Schutzkolloidlösung zu expandieren. Durch diese Art der Abscheidung der Nanopartikel werden das Wachstum und die nachfolgende Agglomeration der Partikel behindert und die Partikel frühzeitig stabilisiert (M. Türk, Chemie Ingenieur Technik (75) 2003, S. 792 - 795).

Als Nachteile der bekannten Verfahren sind zu nennen:
- Alle bei Umgebungsdruck arbeitenden Inertgas-Kondensations- und Desublimationsverfahren erfordern bei schwerflüchtigen Stoffen die Anwendung hoher Temperaturen, um überhaupt nennenswerte Mengen der zu mikronisierenden Stoffe in die Gasphase zu überführen. Bei hohen Temperaturen können aber bereits Zersetzungsvorgänge auftreten. Dies gilt insbesondere für viele organische Substanzen, die deshalb nur bei Temperaturen bis ca. max. 250 °C verdampft werden können. Unter diesen Bedingungen sind aber die erzielbaren Übersättigungen beim Abkühlen oder Vermischen nicht hoch genug, um ein Aerosol mit ausreichend hohen Partikelanzahlkonzentrationen und mit Partikeln im Nanometerbereich zu erzeugen.
- Bei der Erzeugung von Partikeln durch chemische Reaktionen ist man auf reagierende Stoffsysteme beschränkt und kann somit nur ein stark begrenztes Feld von Nanopartikeln auf diese Weise erzeugen.
- Beim mechanischen Zerstäuben können nur Partikel im Mikrometerbereich hergestellt werden, die zumeist auch eine relativ breite Verteilung aufweisen. Um geringere Partikelgrößen zu erreichen, ist dieses Verfahren ungeeignet. Außerdem ist dieses Verfahren nur für Flüssigkeiten anwendbar.
- Die Partikelerzeugung mit überkritischen Fluiden (z. B. RESS) befindet sich noch in der Entwicklung. Sie erfordert die Anwendung hoher Drücke und einen erhöhten technischen Aufwand beim Einbringen und Ausschleusen der Stoffsysteme. Nach derzeitigen Erkenntnissen können diese Verfahren nur zur Erzeugung von Nanopartikeln aus hochwertigen pharmazeutischen Wirkstoffen wirtschaftlich betrieben werden.
- Die notwendigen Maschinen und Apparate sind mechanisch aufwändig, teuer und wartungsintensiv.

Nachteilig an den aus dem Stand der Technik bekannten Herstellverfahren, welche sich eines organischen Lösungsmittels bedienen, ist die häufig schwierige Isolierbarkeit der Nanopartikel. Außerdem ist es häufig nicht möglich, die Nanopartikel vollständig vom organischen Lösungsmittel zu befreien, was insbesondere kosmetische und pharmazeutische Anwendungsmöglichkeiten stark einschränkt.

Ein weiterer Nachteil von Herstellverfahren des Stands der Technik besteht darin, dass bei der Herstellung als Beiprodukte entstehende Salze oft nur schwer entfernbar sind.

Der Erfindung liegt daher die Aufgabe zugrunde, ein Herstellungsverfahren der eingangs genannten Art zu entwickeln, mit welchem aus schwerflüchtigen Substanzen Nanopartikel mit einer hohen Partikelanzahlkonzentration herstellbar sind, so dass ein nachfolgendes Aufkonzentrieren nicht notwendig oder auf einfache Weise durchzuführen ist. Die Herstellung soll unter milden Temperatur- und Druckbedingungen und mit geringem technischem Aufwand hinsichtlich der mechanischen Anforderungen der Anlage, der Apparatekosten und deren Wartung erfolgen.

Diese Aufgabe wird im eingangs genannten Verfahren zur Herstellung von Nanopartikeln aus einer oder mehreren Substanzen, insbesondere aus Lipiden, dadurch gelöst, dass man die Substanz in die Gasphase überführt, die Gasphase komprimiert und gleichzeitig abkühlt und die Mikro- bzw. Nanopartikel aus dem erzeugten Aerosol abscheidet.

Die Abscheidung der Nanopartikel kann in bekannter Weise erfolgen, z. B. in einem Venturi-Wäscher, in einem Elektro-Nassfilter oder in einer Blasensäule. Dort wird das Aerosol vorzugsweise von unten in einen die Schutzkolloidlösung enthaltenden Behälter eingeleitet.

Der besondere Vorteil des erfindungsgemäßen Verfahrens liegt in der Möglichkeit, die erzeugten Nanopartikel beim Abscheiden in eine beliebige Flüssigphase einzubringen. Im Gegensatz zu den bekannten Verfahren wird bei diesem Verfahren der Schritt der Partikelstabilisierung durch Schutzkolloide und Emulgatoren getrennt vom Schritt der Partikelerzeugung durchgeführt. Dadurch können Schutzkolloide und/oder Emulgatoren genau für den Zweck der Stabilisierung ausgewählt werden. Erfindungsgemäß ist man somit durch die Herstellung der Nanopartikel nicht wie im Stand der Technik auf vorgegebene Flüssigphasen angewiesen.

Besonders bevorzugt werden mit dem erfindungsgemäßen Verfahren Nanopartikel aus Wachsen hergestellt, wie sie in der WO 01/39 729 A1 (Cognis Deutschland GmbH) definiert und im Einzelnen beschrieben sind. Zur Vervollständigung der Offenbarung wird ausdrücklich auf die in dieser Schrift genannten Wachse und wachsartigen Substanzen verwiesen. Zur Verwendung der mit dem erfindungsgemäßen Verfahren hergestellten Nanopartikel wird ebenfalls auf die genannte internationale Patentanmeldung verwiesen. Die dort genannten Verwendungen sind auch für die erfindungsgemäß hergestellten Nanopartikel besonders bevorzugt.

Beim erfindungsgemäßen schlagartigen Komprimieren und Kühlen der die interessierenden Substanzen erhaltenden Gasphase wird diese sehr stark übersättigt und es bilden sich spontan die äußerst kleinen Nanopartikel. Überraschenderweise verbleibt die bei weitem überwiegende Menge der Substanz als Aerosol in der Gasphase. Die Anlage ist vorzugsweise zwischen Verdampfer und Wasserringpumpe so isoliert oder beheizt, dass es nicht zur Kondensation kommt.

Vorzugsweise führt man die Kompression und die Abkühlung der Gasphase in einer Flüssigkeitsringpumpe durch, insbesondere in einer Wasserringpumpe. Die Abkühlung erfolgt durch Verdunstungskühlung. Bei einer Flüssigkeitsringpumpe sitzt ein mit Schaufeln versehenes Laufrad exzentrisch im Gehäuse. Durch die Laufraddrehung bildet die Betriebsflüssigkeit im Gehäuse einen mitumlaufenden Flüssigkeitsring, der sich von der Laufradnabe abhebt. Bei der Drehung des Laufrades wird das einerseits von den Schaufeln und der Nabe und andererseits von dem Flüssigkeitsring eingeschlossene Volumen abwechselnd komprimiert und expandiert, wodurch sich die Pumpwirkung ergibt. An der Stelle der größten Expansion tritt das zu fördernde Gas über den Sauganschluss in den entstandenen Unterdruckbereich der Pumpe ein. Nach einer halben Umdrehung erreicht dieser Raum die Stelle der größten Verdichtung und das Gas wird aus dem Druckanschluss herausgeschoben.

Möglicherweise tritt durch die Prozessführung unter Mitwirkung einer Flüssigkeitsringpumpe der überraschende Effekt ein, dass eine Kondensation der in die Gasphase überführten Substanz an den kalten Apparatewänden trotz der hohen Übersättigung kaum auftritt.

Die erfindungsgemäß erzeugten Nanopartikel können je nach Stoffsystem fest oder flüssig sein. Erfindungsgemäß werden die folgenden besonderen Vorteile erreicht. Durch die gleichzeitig ablaufende Kompression und Kühlung werden im Vergleich zu den Verfahren nach dem Stand der Technik, insbesondere zur oben genannten Inertgas-Kondensation bei Umgebungsdruck hier wesentlich höhere Übersättigungen erreicht und damit eine hohe Anzahlkonzentration und besonders kleine Partikel erzielt. Die Nanopartikel können bei vergleichsweise milden Bedingungen für Temperatur und Druck hergestellt werden. Durch die hohe Beladung des Aerosolstromes an Partikeln entfällt ein eventuelles Aufkonzentrieren in nachfolgenden Prozessschritten. Von Vorteil ist, wenn man die Substanz in ein Trägergas verdampft, wobei nicht nur die Verdampfung aus der flüssigen Phase, sondern auch eine Sublimation eingeschlossen ist. Mit nur sehr wenig Trägergas kann eine große Anzahl an Nanopartikeln erzeugt werden. Das Verfahren kann mit handelsüblichen Flüssigkeitsringpumpen durchgeführt werden. Die Beladung eines Trägergasstromes im Vakuum bei erhöhten Temperaturen erlaubt die Erzeugung von Nanopartikeln auch aus solchen Substanzen, die bei Umgebungstemperatur zu niedrige Dampfdrücke aufweisen.

Als Trägergas sind Stickstoff, Sauerstoff, Luft, Kohlendioxid, Edelgase geeignet. Besonders bevorzugt als Trägergas ist Stickstoff. Allgemein sind als Trägergas sämtliche Gase geeignet, die nicht mit den Substanzen der Nanopartikel chemisch reagieren und die beim Komprimieren und gleichzeitigem Abkühlen im gasförmigen. Aggregatzustand bleiben.

Die Verdampfung, der insbesondere schwer flüchtigen Substanzen erfolgt insbesondere unter Vakuum (Unterdruck) in einem Trägergasstrom bei möglichst geringen erhöhten Temperaturen, vorzugsweise kleiner als 250 °C, so dass eine thermische Belastung der oft temperaturempfindlichen Substanzen nicht eintritt. Zur schonenden Verdampfung wird daher vorgeschlagen, dass man die Substanz bei einem Druck unterhalb von 1 bar verdampft.

Je nach Stoffsystem kann man feste oder flüssige Mikro- bzw. Nanopartikel erzeugen. Die erzeugten flüssigen Mikro- bzw. Nanopartikel sind für die Herstellung von Emulsionen geeignet. Besonders bevorzugt ist hier aber die Herstellung von festen Nanopartikeln aus Wachsen und wachsartigen Substanzen, wie bereits oben ausgeführt worden ist.

Die Erfindung betrifft auch eine Anlage zur Durchführung des genannten Verfahrens. Hierzu wird vorgeschlagen, dass ein Verdampfer mit seinem Auslass an eine kühlbare Kompressionseinheit angeschlossen ist, deren Auslass wiederum mit einer Abscheideeinheit für die erzeugten Mikro- bzw. Nanopartikel verbunden ist. Der Verdampfer dient zum Überführen der interessierenden Substanz in die Gasphase. Dabei kann die Verdampfung aus dem flüssigen oder aus dem festen Aggregatzustand erfolgen. Als Abscheideeinheit können die bereits oben genannten Venturi-Wäscher, Elektronassfilter oder eine Blasensäule verwendet werden.

In einer bevorzugten Ausgestaltung ist der Verdampfer als ein an eine Gasquelle angeschlossener Gassättiger ausgebildet. Unter einem "Gassättiger" ist ein Vorlagebehälter mit der zu verdampfenden Substanz (in flüssiger oder fester Form) zu verstehen. Die Substanz kann mittels einer Heizung auf die gewünschte Temperatur, z. B. oberhalb der Schmelztemperatur gebracht werden. Ein Gasstrom streicht über die Oberfläche der verdampfenden Substanz und sättigt sich mit dieser.

Vorzugsweise ist die Kompressionseinheit als eine Flüssigkeitsringpumpe, insbesondere als eine Wasserringpumpe ausgebildet. Die Kühlung des zu verdichtenden Gases erfolgt hierbei durch ein teilweises Verdampfen der umlaufenden Flüssigkeit und die damit verbrauchte Verdampfungswärme.

Die Erfindung betrifft schfießlich noch die Verwendung der nach diesem Verfahren hergestellten Mikro- bzw. Nanopartikel zur Herstellung von Präparaten zur kosmetischen Behandlung des Körpers (Haut, Haare, Nägel und dergleichen), zur Herstellung von Arzneimitteln und/oder zur Herstellung von Lebensmitteln oder Lebensmittelzusatzstoffen. Eine Reihe von Einsatzmöglichkeiten für die erfindungsgemäßen Stoffe ist oben in dieser Anmeldung aufgeführt, ohne dass dies eine abschließende Aufzählung darstellen würde.

Im Folgenden werden Ergebnisse von Versuchen zum erfindungsgemäßen Verfahren anhand von Zeichnungen näher erläutert. Es zeigen
- Figur 1: ein Fließbild eines Versuchsaufbaus und
- Figur 2: eine schematische Darstellung einer Abscheideeinheit für die erzeugten Nanopartikel, die in Kombination mit dem Aufbau nach Figur 1 zur Herstellung von Suspensionen bzw. Emulsionen mit den erfindungsgemäßen Nanopartikein einsetzbar ist.

In allen Zeichnungen haben gleiche Bezugszeichen die gleiche Bedeutung und werden daher gegebenenfalls nur einmal erläutert.

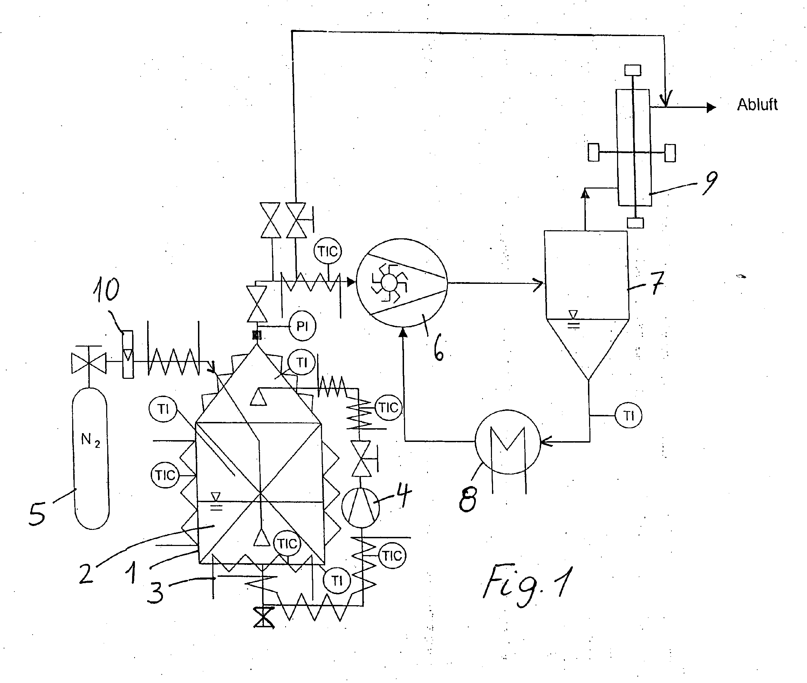

Die Anlage nach Figur 1 bestand im Wesentlichen aus drei Teilen:

Der erste Teil war ein Gassättiger 1 (Vorlagebehälter, Durchmesser = 20 cm, Höhe = 50 cm), der mit flüssigem Lipid 2 gefüllt war. Zur Erhöhung der Stoffaustauschfläche dienten Füllkörper aus Keramik. Mittels einer Heizung 3 konnte der Gassättiger 1 bis auf eine Temperatur von 200 °C erhitzt werden. Das flüssige Lipid wurde mit Hilfe einer Kreiselpumpe 4 aus Edelstahl umgepumpt. Ein Stickstoffstrom aus einer Gasflasche 5 wurde im Gegenstrom im Vakuumbehälter 1 mit dem Lipid aufgesättigt.

Der zweite Teil der Versuchsanlage bestand aus einer Wasserringpumpe 6 vom Typ LEMA 91 AZ AAE und einem Standabscheider 7 vom Typ XBP 0413 der Fa. SIHI Siemen und Hinsch GmbH, Itzehoe. Das Saugvermögen der Wasserringpumpe 6 lag zwischen 24-57 m³/h bei einem Ansaugdruck von 33 - 900 mbar.

Der Aerosolstrom entstand durch gleichzeitige Abkühlung und Kompression des eintretenden Gas-Dampf-Gemisches, wodurch sehr hohe Sättigungsgrade erreicht wurden. Mit einem Kühler 8 wurde das Betriebswasser der Wasserringpumpe 6 auf der gewünschten niedrigen Temperatur gehalten.

In der sich anschließenden Messstrecke (dritter Teil der Versuchsanlage) wurde mit einem Drei-Wellenlängen-Extinktions-Messgerät 9 (3-WEM) vom Typ WIZARD-DQ der Fa. WIZARD Zahoransky KG, Todtnau (Schwarzwald) der Lipidnebel bezüglich seines mittleren Durchmessers und der Anzahlkonzentration charakterisiert. Diese online- und in-situ-Messtechnik beruht auf dem Prinzip der Abschwächung der Intensität dreier monochromatischer Lichtstrahlen unterschiedlicher Wellenlängen beim Durchgang durch ein Partikelkollektiv. Bei angenommener Normalverteilung der Partikelgröße und bekanntem Brechungsindex der Partikel wurde aus den gemessenen Extinktionsverhältnissen mit Hilfe der Mie-Theorie sowohl der mittlere Durchmesser als auch die Anzahlkonzentration der Partikel bestimmt.

Die gesamte Versuchsanlage war mit Heizbändern und entsprechenden Regeleinheiten ausgestattet, so dass eine einheitliche Thermostatisierung gewährleistet war. Der zu beladene Stickstoffstrom wurde mittels eines Rotameters 10 bestimmt.

In einem Versuchsbeispiel zum erfindungsgemäßen Verfahren wurden Nanopartikel aus einem C16-Fettalkohol ("Lanette 16", Produkt der Firma Cognis Deutschland GmbH & Co. KG, Düsseldorf) hergestellt. Der Fettalkohol wurde bei 80 °C aufgeschmolzen und in den ebenfalls auf 80 °C vortemperierten Vakuumbehälter 1 eingefüllt. Der als Verdampfer wirkende Vakuumbehälter 1 und die Rohrleitungen zur Wasserringpumpe 6 wurden dann auf eine Versuchstemperatur von 90 °C und in einem weiteren Versuch auf eine Temperatur von 110 °C gebracht. Ein Stickstoffstrom von 7 Nm³/h wurde im Vakuumbehälter 1 bei einem Druck von 70 mbar mit dem Lipid gesättigt. Das gesättigte Gas-Dampf-Gemisch wurde von der Wasserringpumpe 6 angesaugt und dort komprimiert und gleichzeitig auf Umgebungstemperatur (etwa 20 °C) abgekühlt. Eine starke Nebelbildung, offensichtlich durch eine homogene Keimbildung war die Folge. Das auf diese Weise erzeugte Aerosol strömte über den Abscheider 7 in eine Glaszelle des Drei-Wellenlängen-Extinktions-Messgerätes 9 mit einer optischen Wellenlänge von 85 cm. Mittels des Messgerätes 9 wurden der mittlere Durchmesser und die Anzahlkonzentration der erzeugten Partikel bestimmt.

Es wurden zwei Versuche mit einer Temperatur der Versuchsanlage von 90 °C und von 110 °C durchgeführt. Eine Konzentration der Nanopartikel zwischen 10⁷ und 10⁸ cm⁻³ bei einem mittleren Durchmesser unterhalb von 500 nm wurde in beiden Versuchen erhalten.

Die Versuche haben gezeigt, dass es erfindungsgemäß möglich ist, sehr dichte und feine Aerosole, auch mit Substanzen mit niedrigen Dampfdrücken von etwa 8 Pa bei einer relativ niedrigen Versuchstemperatur von etwa 100 °C herzustellen. Entscheidend für die Herstellung ist der Druck bzw. das Kompressionsverhältnis in der Pumpe 6, wobei die Temperatur eine eher untergeordnete Rolle spielt.

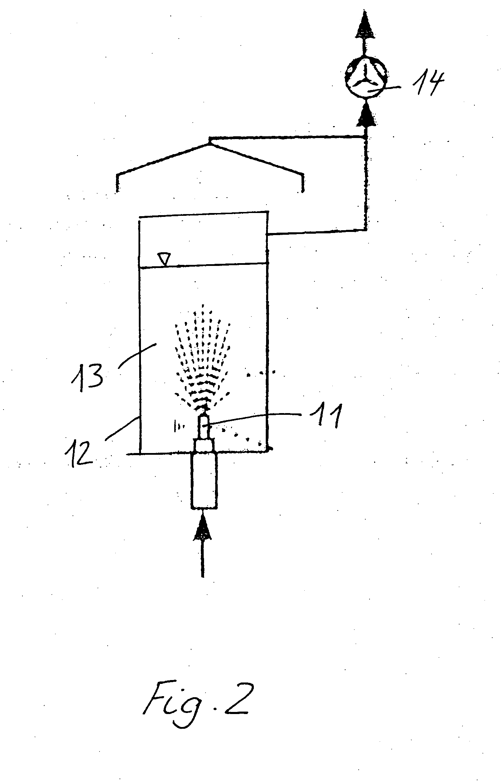

Zur Abscheidung der erzeugten Nanopartikel wird anstelle des Messgerätes 9 in Figur 1 die Anordnung nach Figur 2 angeschlossen. Hier strömt das Aerosol durch eine Düse 11 in einen mit einer Schutzkolloidlösung 13 gefüllten Behälter 12. Anstelle einer Düse 11 kann auch eine einfache Öffnung mit einem größeren Öffnungsquerschnitt vorgesehen sein. Die Abluft, die im Wesentlichen aus dem Inertgas, in diesem Fall also aus Stickstoffgas besteht, wird von einer Pumpe 14 abgesaugt. Im Behälter 12 erhält man dann die gewünschte Suspension bzw. Emulsion der Nanopartikel.

Das erfindungsgemäße Verfahren lässt sich vorzugsweise für Wachse und wachsartige Substanzen, z. B. feste Fettalkohole, aber auch andere Fettderivate einsetzen, die vorzugsweise einen Schmelzpunkt zwischen 30 und 150 °C haben. Die erzeugten Nanopartikel lassen sich verwenden zur Herstellung von Shampoos, Hautcremes, Sonnenschutzmitteln und vielen anderen, in dieser Anmeldung genannten oder darauf verwiesenen Produkten. Mit den erzeugten Nanopartikeln lassen, sich wässrige und nichtwässrige Suspensionen herstellen. Dabei liegt der besondere Vorteil in der Möglichkeit, die Nanopartikel in eine beliebige Fiüssigphäse abzuscheiden, ohne dass man wie im Stand der Technik je nach den eingesetzten Nanopartikeln auf bestimmte Flüssigphasen festgelegt ist. Besonders vorteilhaft ist die hohe Partikelkonzentration, die zwischen 10⁵ und 10⁸ Teilchen pro cm³ liegt. Die erzeugten Nanopartikel können in fester oder flüssiger Form erzeugt werden. Bei Verwendung einer Flüssigkeitsringpumpe erfolgt die gleichzeitig mit der Kompression vorzunehmende Abkühlung durch eine Teilverdampfung der Ringflüssigkeit in der Pumpe, die in der Regel eine Wasserringpumpe ist. Durch das gleichzeitige Ablaufen von Abkühlung und Kompression wird eine besonders hohe Übersättigung in der Gasphase und damit eine hohe Partikelkonzentration erzeugt.

### Bezugszeichenliste

- 1: Gassättiger (Vakuumbehälter)
- 2: Lipid
- 3: Heizung
- 4: Kreiselpumpe
- 5: Gasflasche
- 6: Wasserringpumpe
- 7: Standabscheider
- 8: Kühler
- 9: Drei-Wellenlängen-Extinktions-Messgerät
- 10: Rotameter (Durchflussmessgerät)
- 11: Düse
- 12: Behälter
- 13: Schutzkolloidlösung
- 14: Pumpe

## Patentansprüche

1. Verfahren zur Herstellung von Mikropartikeln oder Nanopartikeln aus einer oder mehreren Substanzen, insbesondere aus Lipiden,
**dadurch gekennzeichnet,**
**dass** man die Substanz in die Gasphase überführt, die Gasphase komprimiert und gleichzeitig abkühlt und die Mikro- bzw. Nanopartikel aus dem erzeugten Aerosol abscheidet.

2. Verfahren nach Anspruch 1,
**dadurch gekennzeichnet,**
**dass** man die Kompression und die Abkühlung der Gasphase in einer Flüssigkeitsringpumpe, insbesondere in einer Wasserringpumpe durchführt.

3. Verfahren nach Anspruch 1 oder 2,
**dadurch gekennzeichnet,**
**dass** man die Substanz in ein Trägergas verdampft.

4. Verfahren nach Anspruch 1, 2 oder 3,
**dadurch gekennzeichnet,**
**dass** man die Substanz bei einem Druck unterhalb von 1 bar verdampft.

5. Verfahren nach einem der vorhergehenden Ansprüche,
**dadurch gekennzeichnet,**
**dass** man feste oder flüssige Mikro- bzw. Nanopartikel erzeugt.

6. Anlage zur Durchführung des Verfahrens nach einem der vorhergehenden Ansprüche,
**dadurch gekennzeichnet,**
**dass** ein Verdampfer (1) mit seinem Auslass an eine kühlbare Kompressionseinheit (6) angeschlossen ist, deren Auslass wiederum mit einer Abscheideeinheit (11, 12, 13) für die erzeugten Mikro- bzw. Nanopartikel verbunden ist.

7. Anlage nach Anspruch 6,
**dadurch gekennzeichnet,**
**dass** der Verdampfer als ein an die Gasquelle (5) angeschlossener Gassättiger (1) ausgebildet ist.

8. Anlage nach Anspruch 6 oder 7,
**dadurch gekennzeichnet,**
**dass** die Kompressionseinheit als eine Flüssigkeitsringpumpe, insbesondere als eine Wasserringpumpe (6) ausgebildet ist.

9. Verwendung der nach dem Verfahren nach einem einem der Ansprüche 1 bis 5 hergestellten Mikro-bzw. Nanopartikel zur Herstellung von Präparaten zur kosmetischen Behandlung des Körpers (Haut, Haare, Nägel und dergleichen), zur Herstellung von Arzneimitteln, und/oder zur Herstellung von Lebensmitteln oder Lebensmittelzusatzstoffen.

10. Suspension oder Emulsion enthaltend Mikro- bzw. Nanopartikel, hergestellt nach dem Verfahren nach einem der Ansprüche 1 bis 5.
